# EUROPEAN PATENT APPLICATION

(11) **EP 3 603 556 A1**
(43) Date of publication of application: **05.02.2020**
(21) Application number: 19197531.7
(22) Date of filing: 27.02.2018
(51) Int. Cl.: A61B 18/14, A61B 90/00, A61B 18/00

(54) **LENS IN BALLOON CATHETER**

(30) Priority: 28.02.2017 US 201715444521
(62) Divisional of application: 18158811.2
(71) Applicant: Biosense Webster (Israel) Ltd., Yokneam, 2066717 (IL)
(72) Inventor: GLINER, Vadim, 2066717 Yokneam (IL)
(74) Representative: Small, Gary James

(57) **Abstract**

An apparatus includes a probe having a lumen and a first inflatable transparent balloon deployable through the lumen. The first balloon is coupled to be filled with liquid. The apparatus includes a second inflatable transparent balloon, deployable through the lumen, and disposed inside the first inflatable balloon between a proximal side and a distal side of the first inflatable balloon. The second balloon is coupled to be filled with air. The apparatus also includes a camera deployable through the lumen between a proximal side and a distal side of the second inflatable balloon and configured to capture an optical image formed through the first and second inflatable balloons.

## Description

### FIELD OF THE INVENTION

The present invention relates generally to catheters, and specifically to a camera attached to a balloon catheter.

### BACKGROUND

Balloon catheters are used for tissue ablation by having electrodes on the surface of an inflated balloon contacting the tissue, such as cardiac tissue, to be ablated. Once contact has been established, current is passed between the electrodes and the tissue, the heat generated by the current ablating the tissue.

U.S. Patent 8,760,767, which is incorporated herein by reference, describes a non-round fluid lens assembly, which includes a non-round rigid lens and a flexible membrane attached to the non-round rigid lens, such that a cavity is formed between the non-round rigid lens and the flexible membrane.

U.S. Patent 7,929,218, which is incorporated herein by reference, describes a lens that includes a container, a first dielectric fluid that is held in the container, a second dielectric fluid that is held in the container, and a phase boundary layer between the first and the second fluid.

U.S. Patent 7,083,614, which is incorporated herein by reference, describes a collapsible ultrasonic reflector, which incorporates a gas-filled reflector balloon, a liquid-filled structural balloon with an ultrasonic transducer disposed within the structural balloon.

U.S. Patent 7,326,201, which is incorporated herein by reference, describes a collapsible ultrasonic reflector, which incorporates a gas-filled reflector balloon, a liquid-filled structural balloon with an ultrasonic transducer disposed within the structural balloon.

U.S. Patent 7,540,846, which is incorporated herein by reference, describes an apparatus and methods for ablating tissue surrounding a tubular anatomical structure such as the wall of a blood vessel or prostatic tissue surrounding the urethra.

U.S. Patent 8,475,442, which is incorporated herein by reference, describes an ultrasonic assembly suited for attachment to a catheter, e.g. for medical treatment.

U.S. Patent 9,186,485, which is incorporated herein by reference, describes a balloon catheter for delivering a therapeutic and/or diagnostic agent to tissue, including an outer balloon having a wall with an opening therethrough and an inner balloon disposed in the outer balloon.

U.S. Patent Application 20040039242, which is incorporated herein by reference, describes a light source that emits electromagnetic radiation having wavelengths within the visible spectrum.

U.S. Patent Application 20060058711, which is incorporated herein by reference, describes an apparatus and methods for ablating tissue surrounding a tubular anatomical structure such as the wall of a blood vessel or prostatic tissue surrounding the urethra.

U.S. Patent Application 20060155269, which is incorporated herein by reference, describes an apparatus for treating cardiac arrhythmias.

U.S. Patent Application 20100256629, which is incorporated herein by reference, describes methods and devices for treatment of the ostium.

U.S. Patent 6,532,387, which is incorporated herein by reference, describes a catheter device/method for enhancing local administration of pharmaceutical compounds.

### SUMMARY

Embodiments of the present invention that are described hereinbelow provide for an increased field of view for a camera deployed in a balloon catheter.

There is therefore provided, in accordance with an embodiment of the present invention, an apparatus, including a probe having a lumen, a first inflatable transparent balloon deployable through the lumen and coupled to be filled with liquid, a second inflatable transparent balloon deployable through the lumen, disposed inside the first inflatable balloon between a proximal side and a distal side of the first inflatable balloon, and coupled to be filled with air, and a camera deployable through the lumen between a proximal side and a distal side of the second inflatable balloon and configured to capture an optical image formed through the first and second inflatable balloons.

In an embodiment an optical surface provided by the second inflatable transparent balloon increases a field of view (FOV) of the camera.

In another embodiment the first and second inflatable transparent balloons are deployed inside a heart.

In yet another embodiment the first inflatable transparent balloon is partially covered with a multiplicity of electrodes that are configured for cardiac ablation. Typically, the optical image is configured to determine a degree of contact of the first inflatable transparent balloon with a tissue of a heart. Additionally, a selected one of the multiplicity of electrodes may be energized for the cardiac ablation in response to the degree of contact.

In a disclosed embodiment the proximal side of the second inflatable transparent balloon is coincident with the proximal side of the first inflatable transparent balloon. Additionally, the camera may be coincident with the proximal side of the second inflatable transparent balloon.

In another disclosed embodiment the distal side of the second inflatable transparent balloon is coincident with the distal side of the first inflatable transparent balloon.

In yet another disclosed embodiment a diameter of the first inflatable transparent balloon is in a range 28 mm - 32 mm, and a diameter of the second inflatable transparent balloon is in a range 20 mm - 26 mm.

There is also provided, in accordance with an embodiment of the present invention, a method, including providing a probe having a lumen, deploying a first inflatable transparent balloon through the lumen and filling the first inflatable transparent balloon with liquid, deploying a second inflatable transparent balloon through the lumen inside the first inflatable transparent balloon, disposing the second inflatable transparent balloon between a proximal side and a distal side of the first inflatable transparent balloon, and filling the second inflatable transparent balloon with air, and deploying a camera through the lumen between a proximal side and a distal side of the second inflatable transparent balloon and configuring the camera to capture an optical image formed through the first and second inflatable transparent balloons.

The present invention will be more fully understood from the following detailed description of the embodiments thereof, taken together with the drawings in which:

### BRIEF DESCRIPTION OF THE DRAWINGS

Fig. 1 is a schematic illustration of an invasive medical procedure using apparatus, according to an embodiment of the present invention;
Fig. 2 is a schematic perspective view of a balloon catheter in its inflated state, according to an embodiment of the present invention;
Figs. 3A-B are schematic sectional illustrations of a balloon catheter in its inflated state, according to an embodiment of the present invention; and
Fig. 4 is an illustration of simulated increased FOV half-angle, according to an embodiment of the present invention.

### DETAILED DESCRIPTION OF EMBODIMENTS

### OVERVIEW

Balloon catheters used for ablation of tissue, such as cardiac tissue, may be equipped with a camera inside the balloon. The camera is typically positioned at the proximal side of the balloon, with the axis of symmetry of its field of view (FOV) pointing to the distal side of the balloon. The image collected by the camera through the transparent areas of the walls of the balloon may be used to assess the degree of contact between the balloon and the cardiac tissue, and to turn off electrodes that are not in contact with the tissue. In case the balloon is used, for example, for the ablation of the ostium of a pulmonary vein, the degree to which the balloon seals off the vein may be verified by injecting a contrast agent in the vein upstream from the balloon. A rapid flow of the contrast agent past the balloon is an indication of poor sealing, and the images captured by the camera may be used to assess the flow of the contrast agent and thus the degree of sealing. Further, the images captured by the camera will yield information on the surface properties of the cardiac tissue.

A drawback of cameras available for imaging through the balloon is their limited FOV. Embodiments of the present invention address this drawback by providing an apparatus that comprises a probe having a lumen, and a first inflatable transparent balloon which is deployable through the lumen. The first balloon is coupled to be filled with liquid. The apparatus also comprises a second inflatable transparent balloon which is deployable through the lumen, and which is disposed inside the first inflatable balloon at a proximal side of the first inflatable balloon. The second balloon is coupled to be filled with air. The apparatus further comprises a camera which is deployable through the lumen at the proximal side of the first inflatable balloon and which is configured to capture an optical image formed through the first and second inflatable balloons.

### SYSTEM DESCRIPTION

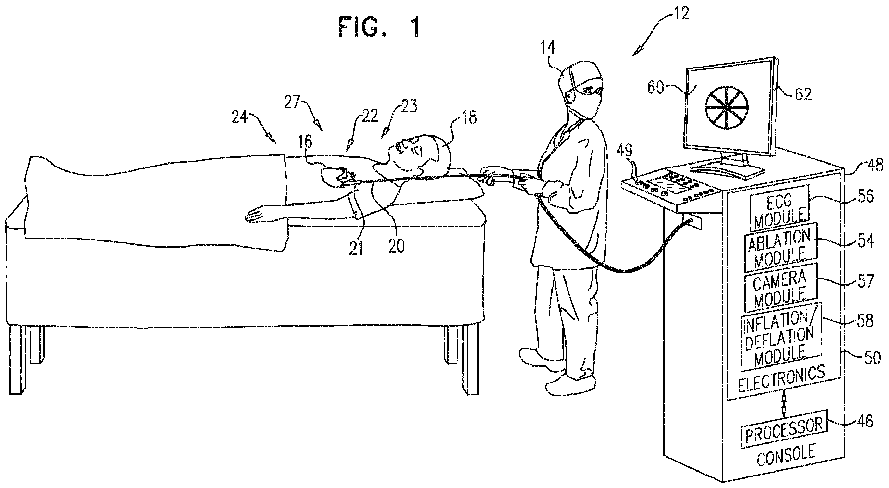

Fig. 1 is a schematic illustration of an invasive medical procedure using apparatus 12, according to an embodiment of the present invention. The procedure is performed by a medical professional 14, and, by way of example, the procedure in the description hereinbelow is assumed to comprise ablation of a portion of a myocardium of a heart 16 of a human patient 18. However, it will be understood that embodiments of the present invention are not just applicable to this specific procedure, and may include substantially any procedure on biological tissue or on non-biological material.

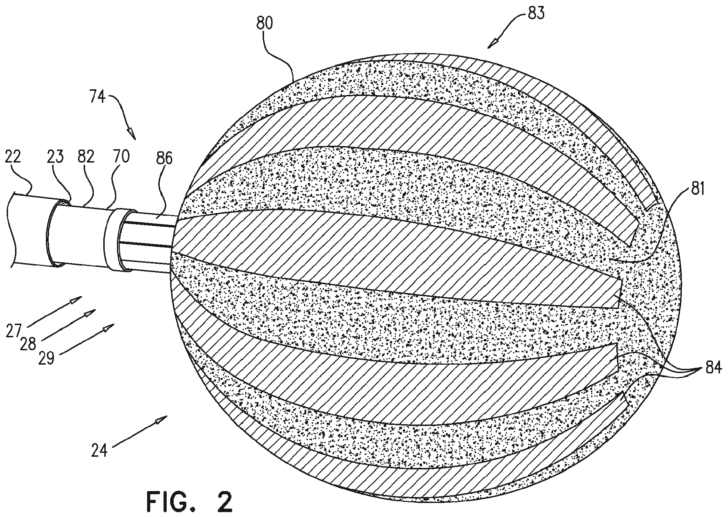

In order to perform the ablation, professional 14 inserts a probe 20 into a sheath 21 that has been pre-positioned in a lumen of patient 18. Sheath 21 is positioned so that a distal end 22 of the probe enters the heart of the patient. A balloon catheter 24 equipped with a camera 27, which is described in more detail below with reference to Figs. 2-3, is deployed through a lumen 23 of probe 20, and exits from the distal end of the probe.

Apparatus 12 is controlled by a processor 46, which is located in an operating console 48 of the apparatus. Console 48 comprises controls 49 which are used by professional 14 to communicate with processor 46. During the procedure, processor 46 typically tracks a location and an orientation of distal end 22 of the probe, using any method known in the art. For example, processor 46 may use a magnetic tracking method, wherein magnetic transmitters external to patient 18 generate signals in coils positioned in distal end 22. The Carto® system produced by Biosense Webster, of Diamond Bar, CA, uses such a tracking method.

The software for processor 46 may be downloaded to the processor in electronic form, over a network, for example. Alternatively or additionally, the software may be provided on non-transitory tangible media, such as optical, magnetic, or electronic storage media.

Professional 14 may use controls 49 to present an image 60, produced by camera 27, on a screen 62.

In order to operate apparatus 12, processor 46 communicates with electronics 50, which have a number of modules used by the processor to operate the apparatus. Thus, electronics 50 comprise an ablation module 54 for controlling ablation power applied, and an electrocardiograph (ECG) module 56 for acquiring electrical signals generated in heart 16. Electronics 50 also comprise a camera module 57, which typically powers camera 27 and which also receives images from the camera, and an inflation/deflation module 58 for inflating and deflating the balloons in balloon catheter 24. Electronics 50 typically comprise other modules, such as a force module for measuring the force on distal end 22, a tracking module for operating the tracking method used by processor 46, and an irrigation module allowing the processor to control irrigation provided for distal end 22. For simplicity, such other modules are not illustrated in Fig. 1. The modules may comprise hardware as well as software elements.

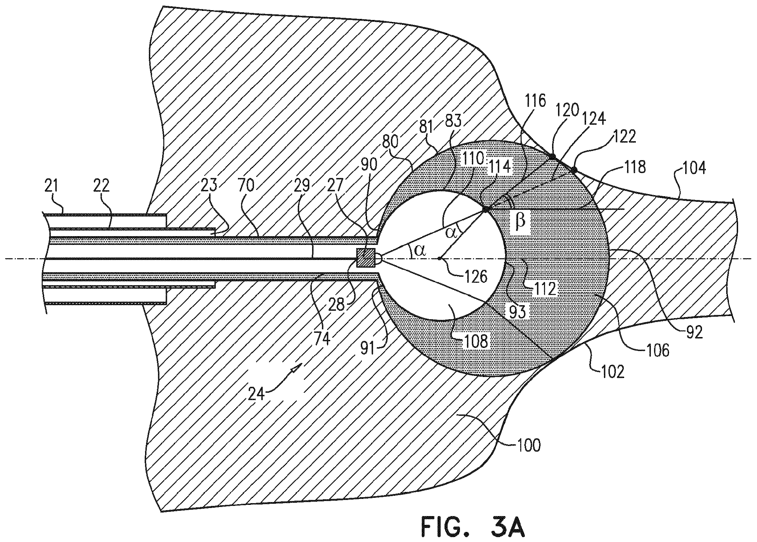

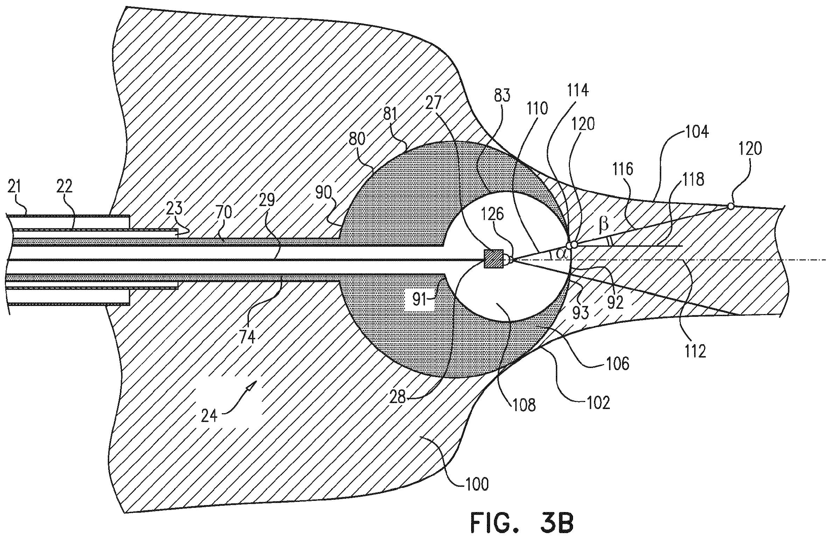

Figs. 2, 3A and 3B are a schematic perspective view and two schematic sectional views, respectively, of balloon catheter 24 in its inflated state, according to embodiments of the present invention. Figs. 3A-B show two alternative embodiments of the present invention, as will be described below. In a disclosed embodiment, where balloon catheter 24 is used to ablate the ostium of a lumen, such as the pulmonary vein, balloon catheter 24 is supported by a tubular shaft 70 having a proximal shaft portion 82. Shaft 70 comprises a hollow central tube 74, which permits a guidewire 29, with camera 27 fixed at a distal end 28 of the guidewire, to traverse the tube.

Balloon catheter 24 comprises two balloons: a first balloon 80 sealed to shaft 70, and a second balloon 83 inside the first balloon, disposed at a proximal side 90 of first balloon 80 and sealed to tube 74. Both balloons are bio-compatible and are typically formed from a plastic such as polyurethane (PU), which is transparent to optical radiation. In operation first balloon 80 and second balloon 83 may be deployed, in an uninflated state, via lumen 23 of probe 20, and after exiting from distal end 22, the balloons may be inflated. First balloon 80 has an external wall 81, over which generally similar electrodes 84 are typically attached by biocompatible cement. Conductive proximal elements 86 of electrodes 84 are coupled to proximal shaft portion 82, and are used by ablation module 54 to provide ablation power to the electrodes. First balloon 80 and electrodes 84 typically have irrigation holes penetrating the balloon and the electrodes, but for simplicity such holes are not shown in Figs. 2, 3A and 3B. The parts of external wall 81 of first balloon 80 that are not covered by electrodes 84 provide camera 27 with transparent windows through which the camera may collect images from outside the first balloon.

Typical steps leading to the initiation of an ablation procedure on a pulmonary vein utilizing balloon catheter 24 in the disclosed embodiment are as follows: a distal end of sheath 21 is positioned in a left atrium 100 of the heart of human patient 18, and probe 22 is inserted to the distal end of the sheath. Shaft 70 is inserted into probe 22 and the probe is guided until the distal end of the shaft is in proximity to the vein. Shaft 70 with balloon catheter 24 is then pushed out of probe 22 and first balloon 80 and second balloon 83 are inflated. First balloon 80 is inflated by filling it with a liquid 106 such as saline solution, and second balloon 83 is inflated by filling it with air 108. As shown in Fig. 3A, at this point proximal side 90 of first balloon 80 and a proximal side 91 of second balloon 83 are coincident. Guidewire 29, together with camera 27 fixed to distal end 28 of the guidewire, is pushed in tube 74 until the camera reaches proximal side 90 of first balloon 80.

When the camera is in place, the inflated balloons and the camera may be pushed further until first balloon 80 contacts an ostium 102 of a pulmonary vein 104. At this stage, camera module 57 may present images of the ostium acquired by camera 27 on screen 62, enabling professional 14 to determine a degree of contact of the first balloon, and its attached electrodes 84, with the ostium. If necessary, using the images acquired by camera 27, professional 14 may adjust the position of first balloon 80 to achieve satisfactory contact, i.e., have a high degree of contact, of the electrodes with the ostium.

Once professional 14 decides that electrodes 84 are in contact with ostium 102, ablation may be initiated. In some cases, the images acquired by camera 27 may indicate to professional 14 that some of electrodes 84 are not in satisfactory contact with the ostium, i.e., have a low degree of contact, and in such an event the professional may decide not to ablate with these electrodes.

The schematic sectional illustration of Fig. 3A is utilized to show the effect of second balloon 83 on the capture of optical images by camera 27 through balloons 80 and 83, while the proximal sides 90 and 91 of balloons 80 and 83, respectively, coincide and camera 27 is located at the common proximal sides of the two balloons. Although first balloon 80 is shown in Fig. 3A with a spherical shape, it may be deformed to a non-spherical shape by being pushed against ostium 102, thus reducing its dimensions from its typical diameter of 28 mm - 32 mm. However, in an embodiment of the present invention the diameter of second balloon 83 may be selected to be sufficiently small, typically 20 mm - 26 mm, so that it will retain its spherical shape even with first balloon 80 deformed by external forces.

The impact of the refraction of optical rays at the air/liquid transition at the surface of second balloon 83 is schematically illustrated by tracing a maximal chief ray 110, originating in camera 27. Herein "chief ray" means a ray that passes through the aperture stop of camera 27, and "maximal" indicates that an angle α that chief ray 110 forms with an optical axis 112 is the largest possible chief ray angle for the camera. Thus angle α is the half-angle of the FOV of camera 27. Optical axis 112 is defined by camera 27 and second balloon 83 as their joint axis of symmetry. Following common practice in geometrical optics, chief ray 110 is traced outwards from the camera. Chief ray 110 impinges on the surface of second balloon 83 at a point 114, and refracts at that point to a ray 116. Ray 116 forms an angle β with a line 118 that is parallel to optical axis 112, propagates next in liquid 106 to a point 120 on first balloon 80, and from there to ostium 102. There is no further significant refraction of ray 116 when it passes across wall 81 of first balloon 80, as the refractive index of the blood (or possibly contrast agent) outside first balloon 80 is close to that of liquid 106. The walls of both balloons are sufficiently thin, typically 20 µm - 100 µm, so that the effect of the walls on the optical rays traversing these walls may be neglected.

If second balloon 83 were not deployed, and if the maximal chief ray angle of camera 27 is still α, ray 110 would propagate to a point 122 as shown by a dotted line 124, and camera 27 would have a smaller FOV half-angle of α instead of β.

Fig. 3B shows a schematic sectional illustration of balloon catheter 24 in its inflated state according to an alternative embodiment of the present invention. Apart from the differences described below, the schematic sectional illustration shown in Fig. 3B is generally similar to the schematic sectional illustration shown in Fig. 3A, and elements indicated by the same reference numerals in both Figs. 3A and 3B are generally similar in construction and in operation. In the disclosed embodiment, guidewire 29 and attached camera 27 are pushed further into second balloon 83, and second balloon 83 is pushed further into first balloon 80. While this repositioning reduces the FOV of the camera from the value achieved when camera 27 is located at proximal side 91 of second balloon 83, there is a corresponding increase of the magnification of camera 27. Fig. 3B shows camera 27 positioned at (or near) center 126 of second balloon 83, while the second balloon is positioned so that a distal side 92 of first balloon 80 and a distal side 93 of second balloon 83 are coincident.

Chief ray 100 again impinges on the surface of second balloon 83 at a point 114, and exits from the second balloon as ray 116. However, due to the fact that camera 27 is located at center 126 of second balloon 83, no (or very little) refraction takes place at point 114, and half-angle β is practically equal to half-angle α. Ray 116 propagates next a very short distance in liquid 106 to a point 120 on first balloon 80, which practically coincides with point 114, and from there to point 120 on pulmonary vein 104. Positioning camera 27 further into second balloon 83 has decreased the FOV of the camera (increased its magnification), and positioning the second balloon towards distal side 92 of first balloon 80 has moved the area imaged to camera 27 deeper into pulmonary vein 104.

From consideration of Figs. 3A and 3B it will be understood that camera 27 can be repositioned to various locations within second balloon 83, and that second balloon 83 can be repositioned to various locations within first balloon 80, and that Fig. 3A and Fig. 3B illustrate but two possible locations for the camera and the two balloons. Thus, repositioning of camera 27 within second balloon 83, and repositioning of second balloon 83 within first balloon 80, provide the ability to adjust both the FOV of the camera (and its magnification) and the images formed by the camera in a continuous fashion.

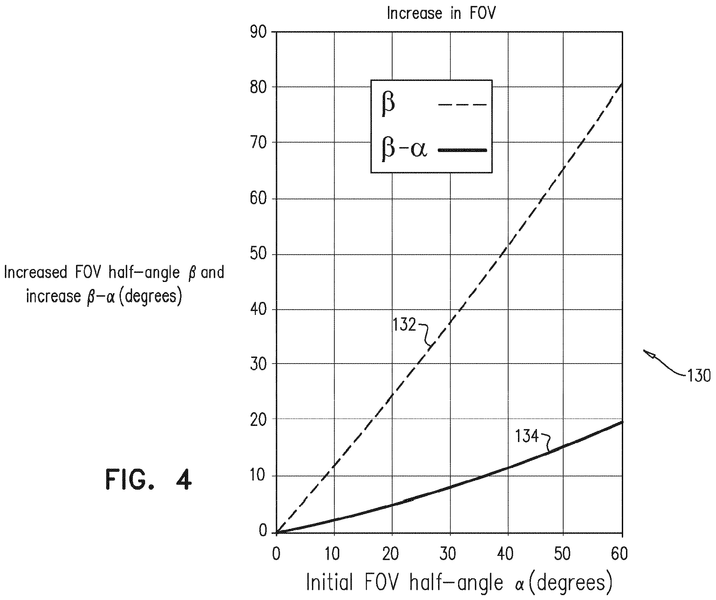

Fig. 4 shows a graph of simulated increased FOV half-angle β vs. an initial half-angle α, according to an embodiment of the present invention. The graph refers to the relative positioning of balloons 80 and 83 as well as camera 27 as shown in Fig. 3A. Angle β has been calculated analytically as a function of FOV half-angle α at camera 27 with second balloon 83 deployed and filled with air, using a refractive index of 1.33 for liquid 106 filling first balloon 80. An aperture stop of camera 27, and thus the origin of ray 110, are at the intersection of optical axis 112 and second balloon 83, the triangle formed by the origin of ray 110, point 114 and a center point 126 of second balloon 83 is an isosceles triangle. Consequently, the angle of incidence of distal end of ray 110 on second balloon 83 is also α, and angle β is independent of the diameter of second balloon 83 as long the spherical shape of second balloon is not affected by a deformation of first balloon 80. Curves 130 show both the increased FOV half-angle β as a curve 132 and the increase β-α of the FOV half-angle as a curve 134, both as a function of the initial FOV half-angle α at camera 27. For an initial FOV half-angle α = 45°, the increased FOV half-angle is β = 57.9°, with an increase of β-α = 12.9°. For an initial FOV half-angle α = 60°, the increased FOV half-angle is β = 79.4°, with an increase of β-α = 19.4°.

It will be understood that the increase in field of view described above is effected by the optical surface, provided by the second balloon, which separates the air in the second balloon from the liquid of the first balloon.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

### ASPECTS OF THE INVENTION

1. A method, comprising:
   providing a probe having a lumen;
   deploying a first inflatable transparent balloon through the lumen and filling the first inflatable transparent balloon with liquid;
   deploying a second inflatable transparent balloon through the lumen inside the first inflatable transparent balloon;
   disposing the second inflatable transparent balloon between a proximal side and a distal side of the first inflatable transparent balloon, and filling the second inflatable transparent balloon with air; and
   deploying a camera through the lumen between a proximal side and a distal side of the second inflatable transparent balloon and configuring the camera to capture an optical image formed through the first and second inflatable transparent balloons.
2. The method according to aspect 1, and comprising increasing a field of view (FOV) of the camera with an optical surface provided by the second inflatable transparent balloon.
3. The method according to aspect 1, and comprising deploying the first and second inflatable transparent balloons inside a heart.
4. The method according to aspect 1, and comprising partially covering the first inflatable transparent balloon by a multiplicity of electrodes that are configured for cardiac ablation.
5. The method according to aspect 4, and comprising determining a degree of contact of the first inflatable transparent balloon with a tissue of a heart from the optical image.
6. The method according to aspect 5, and comprising energizing a selected one of the multiplicity of electrodes for the cardiac ablation in response to the degree of contact.
7. The method according to aspect 1, and comprising positioning the proximal side of the second inflatable transparent balloon to be coincident with the proximal side of the first inflatable transparent balloon.
8. The method according to aspect 7, and comprising positioning the camera to be coincident with the proximal side of the second inflatable transparent balloon.
9. The method according to aspect 1, and comprising positioning the distal side of the second inflatable transparent balloon to be coincident with the distal side of the first inflatable transparent balloon.
10. The method according to aspect 1, wherein the diameter of the first inflatable transparent balloon is in a range 28 mm - 32 mm, and a diameter of the second inflatable transparent balloon is in a range 20 mm - 26 mm.
11. Apparatus, comprising:
   a probe having a lumen;
   a first inflatable transparent balloon deployable through the lumen and coupled to be filled with liquid;
   a second inflatable transparent balloon deployable through the lumen, disposed inside the first inflatable balloon between a proximal side and a distal side of the first inflatable balloon, and coupled to be filled with air; and
   a camera deployable through the lumen between a proximal side and a distal side of the second inflatable balloon and configured to capture an optical image formed through the first and second inflatable balloons.
12. The apparatus according to aspect 11, wherein an optical surface provided by the second inflatable transparent balloon increases a field of view (FOV) of the camera.
13. The apparatus according to aspect 11, wherein the first and second inflatable transparent balloons are deployed inside a heart.
14. The apparatus according to aspect 11, wherein the first inflatable transparent balloon is partially covered with a multiplicity of electrodes that are configured for cardiac ablation.
15. The apparatus according to aspect 14, wherein the optical image is configured to determine a degree of contact of the first inflatable transparent balloon with a tissue of a heart.
16. The apparatus according to aspect 15, wherein a selected one of the multiplicity of electrodes is energized for the cardiac ablation in response to the degree of contact.
17. The apparatus according to aspect 11, wherein the proximal side of the second inflatable transparent balloon is coincident with the proximal side of the first inflatable transparent balloon.
18. The apparatus according to aspect 17, wherein the camera is coincident with the proximal side of the second inflatable transparent balloon.
19. The apparatus according to aspect 11, wherein the distal side of the second inflatable transparent balloon is coincident with the distal side of the first inflatable transparent balloon.
20. The apparatus according to aspect 11, wherein a diameter of the first inflatable transparent balloon is in a range 28 mm - 32 mm, and a diameter of the second inflatable transparent balloon is in a range 20 mm - 26 mm.

## Claims

1. Apparatus (12), comprising:
a probe (20) having a lumen (23);
a first inflatable transparent balloon (80) deployable through the lumen and coupled to be filled with liquid;
a second inflatable transparent balloon (83) deployable through the lumen, disposed inside the first inflatable balloon between a proximal side and a distal side of the first inflatable balloon, and coupled to be filled with air; and
a camera (27) deployable through the lumen between a proximal side and a distal side of the second inflatable balloon and configured to capture an optical image formed through the first and second inflatable balloons;
wherein the first inflatable transparent balloon (80) is partially covered with a multiplicity of electrodes (84) that are configured for cardiac ablation.

2. The apparatus (12) according to claim 1, wherein the first (80) and second (83) inflatable transparent balloons are configured for deployment inside a heart.

3. The apparatus (12) according to claim 1, wherein the proximal side (91) of the second inflatable transparent balloon (83) is coincident with the proximal side (90) of the first inflatable transparent balloon (80).

4. The apparatus (12) according to claim 3, wherein the camera (27) is coincident with the proximal side of the second inflatable transparent balloon (83).

5. The apparatus (12) according to claim 1, wherein the distal side (93) of the second inflatable transparent balloon (83) is coincident with the distal side (92) of the first inflatable transparent balloon (80).

6. The apparatus (12) according to claim 1, wherein a diameter of the first inflatable transparent balloon (80) is in a range 28 mm - 32 mm, and a diameter of the second inflatable transparent balloon (83) is in a range 20 mm - 26 mm.
